# EUROPEAN PATENT SPECIFICATION

(11) **EP 0 703 296 B1**
(45) Date of publication and mention of the grant of the patent: **22.07.1998**
(21) Application number: 95108635.4
(22) Date of filing: 03.10.1988
(51) Int. Cl.: C12Q 1/68

(54) **Polynucleotide determination by displacement of a strand on a capture probe**
Nachweis eines Polynukleotids durch Ersätzung einer Kette an einer Fangsonde
Détermination de polynucléotides par substitution d'un brin sur une sonde de capture

(30) Priority: 29.09.1988 US 251152
(43) Date of publication of application: 27.03.1996
(62) Divisional of application: 88309203.3
(73) Proprietor: CHIRON CORPORATION, Emeryville California 94608-2916 (US)
(72) Inventor: Urdea, Michael S., Alamo, California 94507 (US)
(74) Representative: Goldin, Douglas Michael

(56) References cited:
- EP-A- 0 167 238
- EP-A- 0 269 764
- WO-A-87/03911
- WO-A-88/01302

## Description

### Background of the Invention

### 1. Field of the Invention

The ability to synthesize oligonucleotide sequences at will and to clone polynucleotide sequences prepared by synthetic procedures or obtained from naturally occurring sources has greatly expanded the opportunities for detecting the presence of specific sequences in an extended oligonucleotide sequence, e.g., chromosome(s), mixture of sequences, mRNAs, or the like. Interest in specific sequences may involve the diagnosis of the presence of pathogens, the determination of the presence of alleles, the presence of lesions in a host genome, the detection of a particular mRNA or the monitoring of a modification of a cellular host, to mention only a few illustrative opportunities. While the use of antibodies to perform assays diagnostic of the presence of various antigens in samples has seen an explosive expansion in techniques and protocols since the advent of radioimmunoassay, there has been until recently no parallel activity in the area of the DNA probes. Therefore, for the most part, detection of sequences has involved various hybridization techniques requiring the binding of a polynucleotide sequence to a support and employing a radiolabeled probe.

In view of the increasing capability to produce oligonucleotide sequences in large amounts in an economical way, the attention of investigators will be directed to providing for simple, accurate and efficient techniques for detecting specific oligonucleotide sequences. Desirably, these techniques will be rapid, minimize the opportunity for technician error, be capable of automation, and allow for simple and accurate methods of detection. Toward this end, there have already been efforts to provide for means to label oligonucleotide probes with labels other than radioisotopes and for improving the accuracy of transfer of DNA sequences to a support from a gel, as well as improved methods for derivatizing oligonucleotides to allow for binding to a label. There continues to be a need for providing new protocols which allow for flexibility in detecting DNA sequences of interest in a variety of situations where the DNA may come from diverse sources.

### 2. Description of the Prior Art

Meinkoth and Wahl, Anal. Biochemistry (1984) 138:267-284, provide an excellent review of hybridization techniques. Leary, et al., Proc. Natl. Acad. Sci. USA (1983) 80:4045-4049, describe the use of biotinylated DNA in conjunction with an avidin-enzyme conjugate for detection of specific oligonucleotide sequences. Ranki et al., Gene (1983) 21:77-85 describe what they refer to as a "sandwich" hybridization for detection of oligonucleotide sequences. Pfeuffer and Helmrich, J. of Biol. Chem. (1975) 250:867-876 describe the coupling of guanosine-5'-o-(3-thiotriphosphate) to Sepharose 4B. Bauman, et al., J. of Histochem. and Cytochem. (1981) 29:227-237, describe the 3'-labeling of RNA with fluorescers. PCT Application W0/8302277 describes the addition to DNA fragments of modified ribonucleotides for labeling and methods for analyzing such DNA fragments. Renz and Kurz, Nucl. Acids Res. (1984) 12:3435-3444, describe the covalent linking of enzymes to oligonucleotides. Wallace, DNA Recombinant Technology (Woo, S., Ed.) CRC Press, Boca Raton, Florida, provides a general background of the use of probes in diagnosis. Chou and Merigan, N. Eng. J. of Med. (1983) 308:921-925, describe the use of a radioisotope labeled probe for the detection of CMV. Inman, Methods in Enzymol. 34B, 24 (1974) 30-59, describes procedures for linking to polyacrylamides, while Parikh, et al., Methods in Enzymol. 34B, 24 (1974) 77-102, describe coupling reactions with agarose. Alwine, et al., Proc. Natl. Acad. Sci. USA (1977) 74:5350-5354, describe a method of transferring oligonucleotides from gels to a solid support for hybridization. Chu, et al., Nucl. Acids Res. (1983) 11:6513-6529, describe a technique for derivatizing terminal nucleotides. Ho, et al., Biochemistry (1981) 20:64-67, describe derivatizing terminal nucleotides through phosphate to form esters. Ashley and MacDonald, Anal. Biochem. (1984) 140:95-103, report a method for preparing probes from a surface bound template. These references which describe techniques are incorporated herein by reference in support of the preparation of labeled oligonucleotides.

Published International Application No. WO88/01302 describes a nucleic acid probe assay method for determining a nucleic acid analyte employing a solid support carrying an oligonucleotide capture probe and a second labelled oligonucleotide which also hybridizes to the analyte. EP-A 0269764 describes a nucleic acid probe assay in which nucleic acid analyte with hybridized labelled probe is immobilized and a displacer oligonucleotide probe is employed to displace the labelled probe from the immobilized analyte.

The present invention provides a method for detecting the presence of an oligonucleotide sequence of interest in a nucleic acid analyte present in a nucleic acid sample, said method comprising:
combining under hybridizing conditions said nucleic acid analyte with a first polynucleotide capture probe and a second polynucleotide label probe, wherein said first and second probes have oligonucleotide sequences complementary to sequences present in said analyte so as to form first and second duplexes therewith under said hybridizing conditions, wherein said capture probe is bound to a solid support;
introducing a replacement polynucleotide strand selected so as to form a duplex with said capture probe that. is more stable than said first duplex, thereby displacing from said support said label probe and said analyte; and
detecting label free of said support.

This method enables one to distinguish between specific and nonspecific binding of the label. In the circumstance that nucleic acid analyte is affixed to a support, contacted with a complementary labelled probe and duplex formation assayed on the support, the label can and does bind to the support in the absence of analyte. This direct binding of the label to the support is referred to herein as "nonspecific" binding. If any significant amount of nonspecific binding occurs, label will be detected on the support regardless of the presence of analyte, giving false positive results. By contrast, in the present method, label is detected only when the analyte of interest is present, i.e., only "specific" binding is detected, as a result of use of a displacer probe to release analyte/labelled probe from immobilized capture probe/analyte/labelled probe complex.

### Brief Description of the Drawings

Figure 1 illustrates the difference between specific and nonspecific binding of a label to a solid support.

Figures 2A and 2B schematically illustrate the method of the invention wherein specifically bound label is released through a strand replacement technique.

### Detailed Description of the invention

A reagent will be employed which will include a polynucleotide sequence having an oligonucleotide sequence of interest that hybridizes to the nucleic acid analyte. This reagent will be referred to herein as a "capture probe", which in the present method, is bound to the selected solid support. A labelling probe will also be employed, which may or may not include the sequence of interest.

In order to distinguish the varous nucleotide sequences involved, the following terms will be used:
nucleic acid sample - sample suspected of containing a nucleic acid sequence having an oligonucleotide sequence of interest;
nucleic acid analyte - DNA or RNA in said nucleic acid sample having an oligonucleotide sequence of interest;
oligonucleotide sequence of interest - a DNA or RNA sequence which may be all or part of a nucleotide chain, usually at least six bases, more ususally at least about 10 bases, preferably at least about 16 bases, which may be 5kb or more, usually not more than 2kb, which is diagnostic of a property to be detected, where the property may be a gene or sequence diagnostic of a hereditary trait, pathogen, etc.;
polynucleotide sequence - DNA or RNA sequences employed as reagents for detection of the oligonucleotide sequence of interest, which may be labelled or unlabelled, bound or unbound to a support, and may or may not include a sequence complementary to the oligonucleotide sequence of interest.

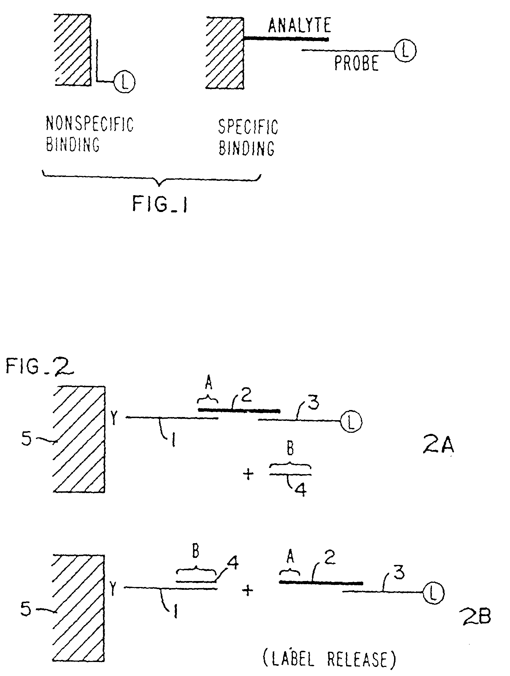

Referring to Figures 2A and 2B, a complex is formed between a capture probe 1 (bound to solid support 5 through linkage Y), the nucleic acid analyte 2, and labelling probe 3. The procedure followed to obtain this hybridization complex is more fully described in EP Publication No. 0225807. In order to release the specifically bound label into solution, a "replacement" polynucleotide strand 4 is introduced, selected so as to form a more stable hybrid with capture probe 1 than the analyte forms with the capture probe. Although G/C content is also a factor, this procedure typically requires that the length "B" of the replacement strand be somewhat longer than the length "A" of the duplex formed between the capture probe and the analyte.

A wide variety of supports and techniques for non-diffusive binding of oligonucleotide chains have been reported in the literature. For a review, see Meinkoth and Wahl, Anal. Biochem. (1984) 138:267-284. Supports include nitrocellulose filters, where temperatures of 80°C for 2 hr suffices, diazotized papers, where bonding occurs without further activation, ecteola paper, etc. Agarose beads can be activated with cyanogen bromide for direct reaction with DNA. (Bauman, et al., J. Histochem. Cytochem. (1981) 29:227-237); or reacted with cyanogen bromide and a diamine followed by reaction with an α-haloacetyl, e.g., bromoacetyl or with an active carboxylic substituted olefin, e.g., maleic anhydride, to provide beads capable of reacting with a thiol functionality present on a polynucleotide chain. For example, DNA can be modified to form an α-thiophosphate for coupling. (Pfeuffer and Hilmreich, J. Biol. Chem. (1975) 250:867-876.) It is also possible to synthesize by chemical means an oligonucleotide bound to a Teflon support and then fully deblock the material without removing it (Lohrmann, et al., DNA (1984) 3:122).

The total number of capture probe components on the support available in the assay medium will vary, for the most part being determined empirically. Desirably, a relatively high concentration per unit surface area of polynucleotide to available functional groups on the support should be employed, so long as the polynucleotide density does not interfere with hybridization.

The size of the polynucleotide will vary widely, usually being not less than about 15 bases and may be 50 bases or more, usually not exceeding about 500 bases, more usually not exceeding 250 bases. There will usually be a region in the polynucleotide reagent component homologous with a sequence in the nucleic acid sample, usually the sequence of interest, of at least six bases, usually at least 12 bases. The region for hybridization may be 16 bases or more, usually not exceeding about lkbp, where perfect homology is not required, it being sufficient that there be homology to at least about 50%, more preferably homology to at least 80%. (By percent homology is intended complementary, ignoring non-complementary insertions which may loop out, insertions being greater than five bases.)

Particularly, where one is interested in a group of allelic genes, a number of different strains, or related species, where the messenger RNA or genomic portion is highly conserved but nevertheless is subject to polymorphisms, it will frequently be desirable to prepare a probe which reflects the differences and optimizes the homology for all the sequences of interest, as against any particular sequence.

A wide variety of labels may be employed for the labelling probe. The label may provide for a detectable signal or means for obtaining a detectable signal. Suitable labels therefore include such diverse substituents as ligands, radioisotopes, enzymes, fluorescers, chemiluminescers, enzyme suicide inhibitors, enzyme cofactors, enzyme substrates, or other substituents which can provide, either directly or indirectly, a detectable signal.

Where ligands are involved, there will normally be employed a receptor which specifically binds to the ligand, e.g., biotin and avidin, 2,4 -dinitrobenzene and anti(2,4-dinitrobenzene)IgG, etc., where the receptor will be substituted with appropriate labels, as described above. In this manner, one can augment the number of labels providing for a detectable signal per polynucleotide sequence.

For the most part, the labels employed for use in immunoassays can be employed in the subject assays. These labels are illustrated in U.S. Patent Nos. 3,850,752 (enzyme); 3,853,914 (spin label); 4,160,016 (fluorescer); 4,174,384 (fluorescer and quencher); 4,160,645 (catalyst); 4,277,437 (chemiluminescer); 4,318,707 (quenching particle); and 4,318,890 (enzyme substrate).

Illustrative fluorescent and chemiluminescent labels include fluorescein, rhodamine, dansyl, umbelliferone, biliproteins, luminol, etc.

Illustrative enzymes of interest include horse radish peroxidase, glucose-6-phosphate dehydrogenase, acetylcholinesterase, β-galactosidase, α-amylase, uricase, malate dehydrogenase, etc. That is, the enzymes of interest will primarily be hydrolases and oxidoreductases. The hybridization can be performed at varying degrees of stringency, so that greater or lesser homology is required for duplexing. For the most part, aqueous media will be employed, which may have a mixture of various other components. Particularly, organic polar solvents may be employed to enhance stringency. Illustrative solvents include dimethylformamide, dimethylacetamide, dimethylsulfoxide, that is, organic solvents which at the amounts employed, are miscible with water. Stringency can also be enhanced by increasing salt concentration, so that one obtains an enhanced ionic strength. Also, increasing temperature can be used to enhance stringency. In each case, the reverse direction results in reduced stringency. Other additives may also be used to modify the stringency, such as detergents.

The period of time for hybridization will vary with the concentration of the sequence of interest, the stringency, the length of the complementary sequences, and the like. Usually, hybridization will require at least about 15 min, and generally not more than about 72 hr, more usually not more than about 24 hr. Furthermore, one can provide for hybridization at one stringency and then wash at a higher stringency, so that heteroduplexes lacking sufficient homology are removed.

The nucleic acid sample will be treated in a variety of ways, where one may employ the intact genome, mechanically sheared or restriction enzyme digested fragments of the genome, varying from about .5kb to 30kb, or fragments which have been segregated according to size, for example, by electrophoresis. In some instances, the sequences of interest will be cloned sequences, which have been cloned in an appropriate vector, for example, a single-stranded DNA or RNA virus, e.g., M13.

Included in the assay medium may be other additives including buffers, detergents, e.g., SDS, Ficoll, polyvinyl pyrrolidone and foreign DNA, to minimize non-specific binding. All of these additives find illustration in the literature, and do not need to be described in detail here.

In accordance with a particular protocol, the sample nucleic acid and polynucleotide reagent(s) are brought together in the hybridization medium at the predetermined stringency. After a sufficient time for hybridization, the support will be washed at least once with a medium of greater or lesser stringency than the hybridization medium.

The subject method can be used for the detection of oligonucleotide sequences, either DNA or RNA, in a wide variety of situations. One important area of interest is the detection of pathogens, viruses, bacteria, fungi, protozoa, or the like, which can infect a particular host. See for example, U.S. Patent No. 4,358,535. Another area of interest is the detection of alleles, mutations or lesions present in the genome of a host, such as involved in amniocentesis, genetic counseling, host sensitivity or susceptibility determinations, and monitoring of cell populations. A third area of interest is the determination of the presence of RNA for such diverse reasons as monitoring transcription, detecting RNA viruses, differentiating organisms through unexpressed RNA, and the like. Other areas of interest, which are intended to be illustrative include monitoring modified organisms for the presence of extrachromosomal DNA or integrated DNA, amplifications of DNA sequences and the maintenance of such sequences.

The physiological samples may be obtained from a wide variety of sources as is evident from the varied purposes for which the subject method may be used.
Sources may include various physiological fluids, such as excreta, e.g., stool, sputum, urine, saliva, etc.; plasma, blood, serum, ocular lens fluids, spinal fluid, lymph, and the like. The sample may be used without modification or may be modified by expanding the sample, cloning, or the like, to provide an isolate, so that there is an overall enhancement of the DNA or RNA and reduction of extraneous RNA or DNA. viruses may be plated on a lawn of compatible cells, so as to enhance the amount of viral DNA; clinical isolates may be obtained by the sample being streaked or spotted on a nutrient agar medium and individual colonies assayed; or the entire sample introduced into a liquid broth and the cells selectively or non-selectively expanded. The particular manner in which the sample is treated will be dependent upon the nature of the sample, the nature of the DNA or RNA source, the amount of oligonucleotide sequence of interest which is anticipated as being present as compared to the total amount of nucleic acid present, as well as the sensitivity of the protocol and label being employed.

The following examples are-offered by way of illustration and not by way of limitation.

### I. Attachment of ribonucleotides to the 3'-end of DNA.

### a. Tailing with terminal deoxynucleotide transferase (TdT).

The following is a modification of the method described by R. Roychoudry, Method in Enzymology (1980) 65:43. A synthetic oligonucleotide of the composition (5' to 3') ATTCGACGCTTATGG (fragment 1) was tailed with rATP. To a solution of 4005 poles of fragment 1 (based on 20 OD₂₆₀ units per mg) in 15 µl of 0.83 nM ATP, 2.5 µl of 10x TdT buffer (1.4 M potassium cacodylate, 0.6 M Tris pH 7.6, 10 MM CoCl₂, 1 mM dithiothreitol (DTT)), 2 µl of TdT (calf thymus, P-L Biochemicals, Inc.; 13.5 units) is added. The 24.5 µl sample was left for 1 hr at 37°C, the evaporated to dryness. The pellet was dissolved in 10 µl of 90% formamide, 0.05% bromophenol blue, 1% Ficoll, heated to 90°C for 5 min and loaded on a 20% denaturing polyacrylamide gel run at 40ma. A band corresponding to fragment 1 extended by one riboadenosine unit was visualized by U.V. shadowing, cut from the gel and eluted overnight in 0.1 M Tris pH 8.0, 5mM EDTA, 0.5m NaCl (Maxam and Gilbert, Methods in Enzymology (1980) 65:499-560). Desalting was achieved with a C-18 SEP-PAK (Waters Associates) as follows: The cartridge was first washed with 5 ml of reagent grade methanol then 10 ml of distilled water. The filtered sample was then applied by syringe to the SEP-PAK. After washing with 20 ml of water, the DNA was eluted with 3 ml of 1:1 (V:V), triethylamine acetate, pH 7.3 : methanol. The solution was then evaporated to dryness (yield ^{∼} 80%).

### b. Ligation with T₄ ligase.

The following process was used to produce a 137 base fragment that contained a 3'-terminal riboadenosine. Fragment 1 from above is used as a "universal" adaptor in order to produce by ligation a ribonucleotide tailed DNA sequence. 
Fragment 2
AGTTGGCAGTACAGCCTAGCAGCCATGGAAACGATGTATATTTCCGCGAGAGGACGACAG
 
Fragment 3
GGTCGTCCGCGGGATTCAGCGCCGACGGACGTAAACAAAGGACGTCCCGCGAAGGATCC
 
Fragment 4
TTCCATGGCTGCTAGGCTGTACTGCCAACTGGATCCTTCGCGGACGTCCTTTGTTTACG
 
Fragment 5
AATTCTGTCGTCCTCTCGCG
 
Fragment 6
CCATAAGCGTCG
 All sequences unless otherwise indicated were 5', 3' hydroxyl. The sequences can be ligated as follows:

5′ 3 2 1 rA 3′
3′ 5′
4 5 6

1) ligation
2) gel isolation

Fragment 2 was 5' phosphorylated with T₄ polynucleotide kinase. To 900 pmoles of the fragment that had been evaporated to dryness were added 2 µl of 10 x KB-TS (500 mM Tris, 100 mM MgCl₂, 10 µg/ml spermidine), 2 µl 10 mM ATP, 2 µl 10 mM DTT, 1 µl (8 units) T₄ kinase (New England Nuclear) and 13 µl of water. After 30 min at 37°C, another 8U of T₄ kinase were added. After an additional 30 min at 37°C, 45 µl (990 pmoles) of fragment 1 that had previously been 5'-phosphorylated in a similar manner were added. After adding 22 µl of 2 M sodium acetate and 8 µl of 250mM EDTA, the solution was heated for 5 min at 65°C to inactivate the T₄ kinase. Fragments 3 through 6 were then added (2.6 µl, 902 pmoles; 8 µl, 1008 pmoles; 32 µl, 1003 pmoles, 45 µl, 936 pmoles, respectively). The solution was vortexed, 680 µl of cold ethanol were added and the solution was placed at -80°C for 20 in. The pellet was then centrifuged at 12,800 RPM for 10 min, decanted, washed with cold ethanol twice and dried.

To anneal the pieces, 18 µl of water were added to dissolve the pellet, the mixture heated in added to dissolve the pellet, the mixture heat in boiling water and cooled to room temperature slowly (^{∼}10 min). At this point were added 3 µl of 10xKB-TS, 3 µl of 10mM ATP and 3 µl of T₄ DNA ligase (New England Biolabs; 40,000 units per ml). After 30 min at 14°C, the solution was dried and purified on a 10% denaturing polyacylamide gel as described above for fragment 1. (Yield ^{∼}75 pmoles.)

### c. Synthesis of DNA on a 2'-nitrobenzyluridine control pore glass support.

The 5'-dimethoxytrityl 2'-nitrobenzyluridine derivative of control pore glass (long chain alkylamino; Pierce Chemical Company) was prepared by Cruachem, Bend Oregon, according to Gough, et al., Tetrahedron Lett. (1981) 22:4177. Oligonucleotide synthesis was carried out on an automatic synthesizer (Warner et al., DNA 3, in press).

The 2'-nitrobenzyl functionality was removed by U.V. irradiation as described by Ohtsuka, et al., Nucleic Acids Res. (1974) 1:1351, except that a 2100 watt mercury bulb was employed. Five min irradiation in a pyrex cuvette was used for all samples (^{∼}14.5 µmoles of 2'-nitrobenzyluridine).

A sequence corresponding to 5' TTCCATGGCTGCTAGGC TGTACTGCCAACTGGATCCTTCGCGGGACGTCCTTTGTTTACGrU 3' (fragment 7) was produced in this manner and used for the coupling described below.

### II. Attachment of DNA by the 3' end to solid supports.

### a. Synthesis of thiosemicarbazido control pore glass (TSC-CPG).

Isothiocyanate control pore glass (Pierce Chemical) was modified with hydrazine to yield the thiosemicarbazido derivative as follows. 400 mg of isothiocyanate CPG was placed in a 50 ml round bottom flask. 25 ml of dimethylsulfoxide, 200 µl of distilled pyridine and 500 µl of a 0.6% hydrazine in dimethylsulfoxide solution were added (see, for example, J. Bauman, et al., J. of Histochem. and Cytochem. (1981) 29:227). After 18 hr with occasional mixing in the dark, the support was washed with 50 ml each of dimethylsulfoxide, pyridine, methanol and 2 L of 0.01 M Tris, pH 7.5.

### b. Attachment of fragment 7 to the solid support.

Approximately 2000 pmoles of fragment 7 was dried from water by evaporation. To this was added 100 µCi of γ³²P-ATP (New England Nuclear), 2 µl of 10xKB (0.5 M Tris HCl pH 7.8, 100 mM MgCl₂, 100 mM DDT), 1 µl (8U) T₄ kinase (New England Nuclear). After 30' at 37°C, the solution was diluted to 1 ml with gel elution buffer and SEP-PAK de-salted as described above. Fragment 7 (20 µl, 982 pmoles) was treated with 20 µl of 1 mM sodium periodate (Sigma) in 0.01 M Tris-HCl, pH 8.0 for 1 hr at 0°C in the dark. To this was added 10 mg of TSC-CPG in 100 µl of 0.1 M sodium acetate, pH 5.6 and the mixture allowed to set for 1 hr at 0°C in the dark, and then at 4°C overnight.

In order to block the remaining thiosemicarbazido functionalities, periodate oxidized ATP was used. A 20 µl sample of 100 mM ATP was treated with 20 mg of sodium periodate in 100 µl of 0.01 M Tris-HCl, pH 8.0. After 1 hr in the dark, 45 µl of the solution was added to the 10 mg of fragment 7-TSC-CPG in 150 µl of 0.1M sodium acetate. After 6 hr at 4°C, the support was washed extensively with 4x standard sodium citrate (SSC).

Based on the incorporated counts, 13% of fragment 7 (128 pmoles) were attached to the glass support.

### III. Attachment of 5' ends of DNA to solid supports.

### a. Preparation of bromoacetyl control pore glass (BA-CPG).

Synthesis of O-bromoacetyl N-hydroxysuccinimide was carried out approximately as described by Cuatreacasas, J. Biol. Chem. (1974) 245:3059.

A mixture of 347 mg of bromoacetic acid and 345 mg of N-hydroxysuccinimide (Sigma) was made up in 20 ml of distilled dioxane. To this mixture was added 532 mg of dicyclohexylcarbodiimide. After 70 min of shaking at room temperature, the cloudy solution was filtered through glass-wool.

To 500 mg of long chain alkylamino control pore glass (Pierce Chemical; 0.1 meq/g) was added 10 ml of 0.10 M sodium phosphate, pH 7.6. The slurry was placed on ice and the O-bromoacetyl N-hydroxysuccinimide solution was slowly added. After 30' with occasional stirring, the BA-CPG was washed with 5L of 0.1 M NaCl.

The number of equivalents of bromoacetate on the support was determined with a 5',5'-dithiobis(2-nitrobenzoic acid) acid (DTNB) test (Butterworth, et al., Arch. Biol. Biophysl. (1967) 118:716). Stock solutions containing 200 mg of DTNB in 50 ml of water and 114 mg of 2-mercaptoethylamine in 100 ml of water were prepared. BA-CPG (10 mg) was reacted with 10 µl of the 2-mercaptoethylamine solution plus 500 µl of 0.05M sodium phosphate at pH 8.0 for 10 min at room temperature. The solution was then tested with 100 µl of DTNB and the visible spectrum was recorded (E=1.36x10⁴ mol⁻¹cm⁻¹ at pH 8). A control was run with 2-mercaptoethylamine without BA-CPG. From the amount of 2-mercaptoethylamine lost upon treatment with BA-CPG, it was determined that BA-CPG contained 10 mmoles bromoacetate per mg.

### b. 5' attachment of DNA to BA-CPG.

To 10 µl (333 pmoles) of fragment 3 (see above) was added 10 µl of 3-³⁵S-ATP (adenosine 5'-0-(3-thiotriphosphate; 0.25mCi, New England Nuclear)), 2-5 µl of 10xKB and 1 µl (8U) of T₄ polynucleotide kinase. After 30 min at 37°C, 1 µl of 50mM 3-S-ATP (lithium salt; P.-L. Biochemicals) and 1 µl (8U) T₄ kinase were added. After an additional 30min at 37°C, the fragment was gel isolated as described above (yield 266 pmoles). Samples were counted on a Beckman LS7000 liquid scintillation counter in Atomlite (New England Nuclear).

A 5 mg sample of BA-CPG was washed by centrifugation 3 times with water and 2 times with 0.10M sodium phosphate, pH 7.6. The 5'thiophosphate fragment 3 was dissolved in 100 µl of the phosphate buffer and added to the washed BA-CPG. The slurry was mixed by rotation on a rotary evaporator for 2 hr at room temperature. The solution was decanted and discarded. In order to block the remaining bromoacetate functionalities, the support was treated with 200 µl of 50mM sodium phosphate, pH 8.0 and 50 µl of 2-mercaptoethanol for an additional 2 hr. Subsequently the solution was decanted and the support was extensively washed with 4XSSC (yield ^{∼}10 pmoles per mg of CPG).

### Iv. Synthesis of Horseradish Peroxidase-DNA Conjugates.

### a. Purification by Elutip.

Horseradish peroxidase (HRP) (2 mg; Type VI, Sigma; 10,000U/38 mg) was dissolved in 0.5 ml of 0.1M sodium phosphate buffer, pH 7.5. O-bromoacetyl N-hydroxysuccinimide (15 µl) was added to the above solution and reaction allowed to proceed for 30 min at room temperature. The solution was passed over a PD-10 sephadex™ G-25M column (Pharmacia) that had previously been equilibrated with 30 ml of 0.1 M sodium phosphate, pH 7.5. The brown fraction (1-1.2 ml) was collected. Fragment 8 (5' to 3', GGTATTGTTGAACAATGTTGTACTTCTATTTG) that had previously been 5'-thiophosphorylated with 3'-³⁵S-ATP as described above and dried (30 pmoles) was taken up in 50 µl of the phosphate buffer. To this thiophosphorylated fragment 8 solution was added the functionalized HRP and the mixture allowed to set for 30 min at room temperature. The mixture was passed over an Elutip-d (Schleicher and Schuell) column. The peroxidase-DNA conjugate is eluted in the void volume (26% of the counts were recovered). A control experiment conducted as described above but using 5'-³²P-phosphate labeled fragment 8 showed less than 0.5% of the counts were eluted under these conditions.

### b. Separation by gel.

A peroxidase conjugate of fragment 9 (5' to 3', TTGAAGAACTACGGTTTGTTGTCTTGTTTCAGAAAGGACTTGCACAAGACCCAAACC) was produced as above except that 360 pmoles of bromoacetyl horseradish peroxidase was combined with 156 pmoles of fragment 9 in 120 µl of 0.025M sodium phosphate, pH 7.5. Instead of passing over an Elutip-d column, the mixture was evaporated to dryness, suspended in 1 µl of 75% glycerol, 10 µl of H₂O and 1 µl of 1% bromophenol blue. This material was then run on a 10% native protein gel. (Lindle, et al., Methods in Enzymol. (1983) 92:309), A control experiment with 5'-³²P-phosphate fragment 9 was also run. The enzyme-DNA conjugate was well resolved from the unconjugated peroxidase as a faster running ³⁵S-labeled species. The gel was stained with 100 ml of 10 mM Tris-HCl, pH 7.5, 50 mM NaCl, 60 µl of 30% H₂O₂ to which was added 60 mg of 4-chloro-1-naphthol dissolved in 20 ml of cold methanol. Since this stain is based on the horseradish peroxidase activity, it was possible to show that the peroxidase-DNA conjugate was itself active. No new active species was produced with the ³²P-fragment 9 control.

### c. Hybridization of DNA-peroxidase to complementary DNA.

5'-Thiophosphorylated fragment 11 (5' to 3', CCAAGAGCTGGTCAAATCTTGAAGCAAACCTACGACAAGTTCGACACCAACATG AGATCTGACGACGCTTTG) was ³²P-labeled as above. A 10% excess of fragment 12 (with reference to fragment 11) was added to the reaction mixture of 5'-thiophosphate fragment 11 plus bromoacetyl peroxidase. The solution was heated to 60°C for 3 min and cooled to room temperature. A control was performed with fragment 12 plus bromoacetyl enzyme without 5'-thiophosphate fragment 11. A gel was run as before and the reaction mixture of fragment 11 plus bromoacetyl enzyme was run as a standard. The lane of fragment 12 plus enzyme and fragment 11 revealed a new slower running band (relative to the fragment 11-peroxidase conjugate) that contained ³²P label. This band was also positive for peroxidase activity. No enzyme positive-³²P labeled band was found for the fragment 12-peroxidase control.

### V. Assay Example

A. An alkaline phophatase probe was prepared as described by Urdea et al., Nucleic Acids Research 16 (11) 4937-4956 (1988). The probe had the sequence (5' to 3') AAGTACGACAACCACATCGGATGACCTCGGATCGACCT*T
where * is the modified nucleotide represented by the structure 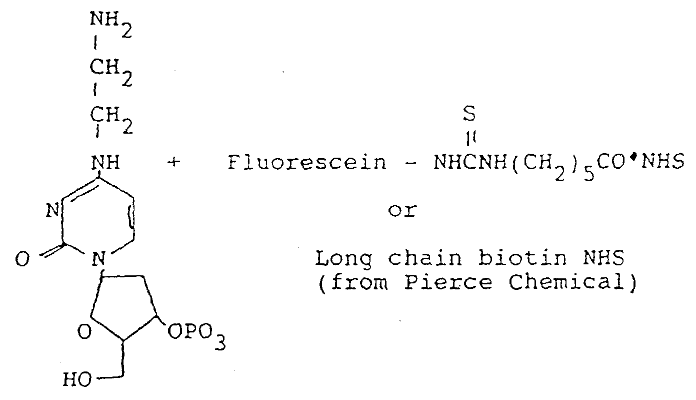 as described in EP Publication No. 0225807

A synthetic oligonucleotide having the sequence (5' to 3') GATGTGGTTGTCGTACTTCTTCTTTGGAGAAAGTGGTG was used as analyte. A second synthetic oligonucleotide was prepared. having the sequence CTTCTTTGGAGAAAGTGGTGTTCATAGAGAAACGATAT ATAGAGACACGATATAGGGATA and was used as the specific release strand, i.e., the replacement strand enabling label release as discussed above. Plates were made using an oligonucleotide having the sequence *TATCCCTATATCGTGT CTCTATATATCGTTTCTCTATGAACACCACTTTCTCCAAAGAAG as capture probe. For this purpose, Microlite I wells (Dynatech) were prepared as follows. To each well, 200 µl of a 200 µg/ml solution of poly-phenylalanyl-lysine (Sigma chemical Inc.) in water was added. The wells were left at room temperature for 30 mins to 2 hours, then washed as above. A 10 OD₂₆₀ sample of the capture probe in 60 µl of 1 X PBS was treated with 140 µl of DMF containing 10 mg of ethylene glycolbis (succinimidylsuccinate) (Pierce Chemicals Inc.). The mixture was vortexed and incubated in the dark at room temperature. After 15 min, the solution was passed over a Sephadex G-25 column (PD-10 from Pharmacia), previously equilibrated with 30 ml of 1 x PBS. The void volume of the column was diluted to a final volume of 35 ml with 1 x PBS. To each well, a 50 µl aliquot of the capture probe solution was added. After covering with plastic wrap, the wells were incubated at room temperature in the dark for 30 min to overnight. After the final incubation step, the wells were washed with 1x PBS, then coated with H buffer, and washed again.

Three sets of capture wells were set up, each set having an analyte-containing well and a control well, i.e., containing no analyte. To each well was added 40 µl 4X SSC with either 1 fmole or no analyte. Hybridization was carried out at 55°C for 1 hour. After washing twice with 380 µl of 4X SSC, 40 µl of 4X SSC containing 100 fmoles of alkaline phosphatase probe were added to the wells. The wells were incubated at 37°C for 1 hr, at which point washing was carried out (1) twice with 380 µl of buffer containing 0.1X SSC and 0.1% SDS, and (2) twice with 380 µl of 4X SSC.

Alkaline phosphatase activity was measured by incubation of the samples with dioxetane, a chemiluminescent substrate. Luminescence was recorded using a microtiter dish reading luminometer (Dynatech).

To one set of wells, 20 µl 4X SSC were added, followed by incubation at 37°C for 1 hr. 20 µl dioxetane were added, and the wells were again incubated at 37°C for 1 hr. Alkaline phosphatase activity was measured, and results are set forth in Table 1 under the heading "No Transfer".

20 µl 4X SSC were added to a second set of wells, which were then incubated at 37°C for 1 hr. The individual solutions were transferred to Microlite I wells, and 20 µl dioxetane was added. The wells were again incubated at 37°C for 1 hr. Alkaline phosphatase activity was measured as above, and results are tabulated in Table 1 under "SSC Release".

20 µl 4X SSC were added to the third set of wells containing 30 pmoles specific release oligonucleotide. The wells were incubated at 37°C for 1 hr, and the solutions were then transferred to Microlite I wells. 20 µl dioxetane were added, and the wells were again incubated at 37°C for 1 hr. Alkaline phosphatase activity was measured as above, and results are set forth in Table 1 under "Oligo Release".

**Table 1**

| Analyte Amount | No Transfer | SSC Release | Oligo Release |
|---|---|---|---|
| 1 fm | 16.26 | 0.47 | 9.52 |
| | 14.66 | 0.56 | 9.89 |
| | 15.76 | 0.55 | 10.42 |
| | | | |
| zero | 0.33 | 0.04 | 0.08 |
| | 0.33 | 0.04 | 0.05 |
| | 0.28 | 0.04 | 0.07 |

| S/N Ratios: | | | |
|---|---|---|---|
| | No Transfer | SSC Release | Oligo Release |
| 1 fmole | 49.66 +/-5.27 | 13.17 +/-1.23 | 149.15 +/-34.84 |

B. The experiment of Section VIII A was repeated, with results set forth in Table 2.

**Table 2**

| Analyte Amount | No Transfer | SSC Release | Oligo Release |
|---|---|---|---|
| 1 fm | 11.82 | 0.20 | 4.05 |
| | 12.39 | 0.18 | 5.02 |
| | 12.72 | 0.19 | 4.79 |
| | | | |
| zero | 0.98 | 0.07 | 0.10 |
| | 1.09 | 0.06 | 0.11 |
| | 1.11 | 0.08 | 0.10 |

| S/N Ratios: | | | |
|---|---|---|---|
| | No Transfer | SSC Release | Oligo Release |
| 1 fmole | 11.6 +/-0.88 | 2.7 +/-0.4 | 46.2 +/-6.9 |

It is evident from the above results, that the subject method provides for a simple, rapid and accurate approach for detecting specific polynucleotide sequences from diverse sources. The method provides for high sensitivity and great flexibility in allowing for different types of labels which involve detectable signals which have been employed in immunoassays. Thus, the subject method can be readily adapted to use in conventional equipment for immunoassays which are capable of detecting radioactivity, light adsorption in spectrophotometers and light emission in fluorometers or scintillation counters. The subject method is applicable to any DNA sequence and can use relatively small probes to reduce false positives and minimize undesirable heteroduplexing.

## Claims

1. A method for detecting the presence of an oligonucleotide sequence of interest in a nucleic acid analyte present in a nucleic acid sample, said method comprising:
combining under hybridizing conditions said nucleic acid analyte with a first polynucleotide capture probe and a second polynucleotide label probe, wherein said first and second probes have oligonucleotide sequences complementary to sequences present in said analyte so as to form first and second duplexes therewith under said hybridizing conditions, wherein said capture probe is bound to a solid support;
introducing a replacement polynucleotide strand selected so as to form a duplex with said capture probe that is more stable than said first duplex, thereby displacing from said support said label probe and said analyte; and
detecting label free of said support.

## Patentansprüche

1. Verfahren zum Nachweis des Vorhandenseins einer Oligonukleotidsequenz von Interesse in einem Nukleinsäureanalyten, der in einer Nukleinsäureprobe vorhanden ist, wobei das Verfahren umfaßt:
Vereinigen des Nukleinsäureanalyten mit einer ersten Polynukleotideinfangsonde und einer zweiten Polynukleotidmarkierungssonde unter Hybridisierungsbedingungen, wobei die ersten und zweiten Sonden Oligonukleotidsequenzen haben, die komplementär zu Sequenzen in dem Analyten sind, so daß mit diesem erste und zweite Duplexe unter den Hybridisierungsbedingungen gebildet werden, wobei die Einfangsonde an einen festen Träger gebunden ist;
Einführen eines Austausch-Polynukleotidstrangs, der so ausgewählt ist, daß ein Duplex mit der Einfangsonde gebildet wird, der stabiler ist als der erste Duplex, wodurch-die Markierungssonde und der Analyt vom Träger verdrängt werden; und
Nachweis nicht an den Träger gebundener Markierung.

## Revendications

1. Une méthode de détection de la présence d'une séquence oligonucléotidique d'intérêt dans un acide nucléique analyte présent dans un échantillon d'acide nucléique, ladite méthode comprenant :
la combinaison dans des conditions d'hybridation dudit acide nucléique analyte avec une première sonde polynucléotidique de capture et une seconde sonde polynucléotidique marqueur, dans laquelle lesdites première et seconde sondes ont des séquences oligonucléotidiques complémentaires des séquences présentes dans ledit analyte de façon à former un premier et un second duplex avec celui-ci dans lesdites conditions d'hybridation, ladite sonde de capture étant liée à un support solide ;
l'introduction d'un brin polynucléotidique de remplacement choisi de manière à former avec ladite sonde de capture un duplex plus stable que ledit premier duplex, déplaçant ainsi ladite sonde marqueur et ledit analyte dudit support ; et
la détection du marqueur non lié audit support.
